# EUROPEAN PATENT SPECIFICATION

(11) **EP 2 247 573 B1**
(45) Date of publication and mention of the grant of the patent: **25.05.2011**
(21) Application number: 09713024.9
(22) Date of filing: 19.02.2009
(51) Int. Cl.: C07C 241/02, C07C 243/40

(54) **CARBONIC AND SULPHURIC ACID SALTS OF 3-(2,2,2-TRIMETHYLHYDRAZINIUM)PROPIONATE ESTERS AND THEIR USE FOR 3-(2,2,2-TRIMETHYLHYDRAZINIUM)PROPIONATE DIHYDRATE PREPARATION**
KOHLENSÄURE- UND SCHWEFELSÄURESALZE VON 3-(2,2,2-TRIMETHYLHYDRAZINIUM)PROPIONATESTERN UND DEREN VERWENDUNG ZUR HERSTELLUNG VON 3-(2,2,2-TRIMETHYLHYDRAZINIUM)PROPIONAT-DIHYDRAT
SELS D'ACIDES CARBONIQUE ET SULFURIQUE D'ESTERS DE 3-(2,2,2-TRIMÉTHYLHYDRAZINIUM)-PROPIONATE ET LEUR UTILISATION POUR LA PRÉPARATION DE DIHYDRATE DE 3-(2,2,2-TRIMÉTHYLHYDRAZINIUM)-PROPIONATE

(30) Priority: 19.02.2008 LV 080022; 19.02.2008 LV 080023
(43) Date of publication of application: 10.11.2010
(73) Proprietor: Grindeks, a joint stock company, Riga 1057 (LV)
(72) Inventor: KALVINS, Ivars, LV-15052 Ikskile (LV); OSVALDS, Pugovics, LV-1004 Riga (LV); CERNOBROVIJS, Aleksandrs, LV-2114 Olaine (LV); IEVINA, Agnija, LV-1004 Riga (LV); LEBEDEVS, Antons, LV-3600 Ventspils (LV)
(86) International application number: PCT/EP2009/051995
(87) International publication number: WO 2009/103772

(56) References cited:
- WO-A-2005/012233
- US-A- 4 481 218
- GILLER S.A. ET AL,.: CHEMISTRY OF HETEROCYCLIC COMPOUNDS, vol. 11, no. 12, 1975, pages 1378-1382, XP002534780

## Description

### Technical Field

The present invention relates to carbonic and sulphuric acid salts of 3-(2,2,2-trimethylhydrazinium)propionate esters and their use for the preparation of 3-(2,2,2-trimethylhydrazinium)propionate dihydrate (international non-proprietary name - "Meldonium").

### Background Art

3-(2,2,2-Trimethylhydrazinium)propionate dihydrate is known for its cardioprotective properties under the International Nonproprietary Name of Meldonium. A number of methods for the preparation of 3-(2,2,2-trimethylhydrazinium)propionate dihydrate are known.

Generally, the process involves 1,1-dimethylhydrazine reaction with acrylic acid esters leading to 3-(2,2-dimethylhydrazino)propionate esters, further alkylated with a methyl halide or dimethylsulphate to give the appropriate methyl-3-(2,2,2-trimethylhydrazinium)propionate halogenide or methylsulphate, which are then hydrolyzed and deionized.

A standard alkaline hydrolysis method of carbonic and sulphuric acid esters in case of the alkyl-3-(2,2,2-trimethylhydrazinium)propionate salts was not successfully realized till now because of the problems of separation of 3-(2,2,2- trimethylhydrazinium) propionate dihydrate and the resulting inorganic salts. It is known that 3-(2,2,2-trimethylhydrazinium)propionate dihydrate forms relatively stable double salts of the variable composition (SU849724). In US 4481218 3-(2,2,2-trimethylhydrazinium) propionate dihydrate is prepared passing a solution of 3-(2,2,2-trimethylhydrazinium) alkyl propionate salts through a ion exchange column.

WO 2008/028514 A (SILVA JORGE) 13.03.2008, disclosed a method of producing the 3-(2,2,2-trimethylhydrazinium) propionate dihydrate by hydrolyzing 3-(2,2,2-trimethylhydrazinium)propionate halide or methyl sulphate esters under acidic conditions, catalyzed by HCl, sulphuric acid, phosphoric acid etc., followed by neutralization by an appropriate inorganic base (for example, sodium, potassium, calcium or magnesium hydroxide or another appropriate base, for example sodium, potassium, lithium or cesium carbonate or bicarbonate etc.) and the double salts thus obtained can be separated by the invented process using saturation of the solution with carbon dioxide or sulphur dioxide.

### Disclosure of Invention

We have unexpectedly found that carbonic and sulphuric acid salts of 3-(2,2,2-trimethylhydrazinium)propionate alky- and arylalkylesters are easily obtainable from the corresponding 3-(2,2-dimethylhydrazino)propionates using dimethylcarbonate (DMC) as the alkylation agent, and are suitable intermediates for preparation of 3-(2,2,2-trimethylhydrazinium)propionate dihydrate. The main advantage of the present invention is the preparation of 3-(2,2,2-trimethylhydrazinium)propionate dihydrate in one step process from carbonic and sulphuric acid salts of 3-(2,2,2-trimethylhydrazinium)propionate alky- and arylalkylesters, unexpectedly without formation of the double salts.

According to the current invention, alkyl-3-(2,2,2-trimethylhydrazinium)propionates of the general formula I: 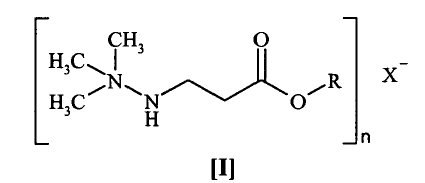 wherein n represents 1 or 2; R represents CH₃, C₂H₅, C₃H₇, i-C₃H₇, C₄H₉, i-C₄H₉ and C₆H₅CH₂; and X represents HCO₃⁻, CO₃²⁻, HSO₄²⁻, or SO₄²⁻,
can easily and nearly quantitatively be hydrolyzed to Meldonium even without using of specific reagents (when X represents HCO₃⁻ and CO₃²⁻) or upon treatment with potassium hydroxide (when X represents HSO₄²⁻ and SO₄²⁻), unexpectedly - without double salts formation.

The method of the present invention may be described in general by sequential combination of the two process steps:
(a) In the first step, alkyl-3-(2,2-dimethylhydrazino)propionate or arylalkyl-3-(2,2,-dimethylhydrazino)propionate or their sulphates were reacted with dimethyl carbonate in methanol. Reaction mixture was heated at 50-115 °C, controlled chromatographically. Methanol or ethanol and dimethyl carbonate excess were removed by distillation at a reduced pressure. The distillation residue was treated with cooled acetone. The solid precipitate was filtered; the crude product was washed on the filter with acetone.
   Recrystallization of crude products yielded 80-96% of the alkyl-3-(2,2,2-trimethylhydrazinium)propionate hydrogen carbonate or arylalkyl-3-(2,2,2-trimethylhydrazinium)propionate hydrogen carbonate (or the corresponding carbonates) and 60-96% of alkyl-3-(2,2,2-trimethylhydrazinium)propionate hydrogen sulphate or arylalkyl-3-(2,2,2-trimethylhydrazinium)propionate hydrogen sulphate (or the corresponding sulphates).
(b) In the second process step Meldonium intermediates of the first step are hydrolyzed into the final product, however for carbonates and sulphates the procedure is slightly different. In the case of alkyl-3-(2,2,2-trimethylhydrazinium)propionate carbonate or arylalkyl-3-(2,2,2-trimethylhydrazinium)propionate carbonate (or the corresponding hydrogen carbonates), concentrated solutions of these substances in water or in water containing solvents were heated with chromatographic control until the full conversion of the starting materials. Depending on the solvent used, reaction temperature can be varied and the corresponding alkanol or arylalkanol, formed as a hydrolysis product, may be simultaneously distilled off the reaction mixture. At the end of reaction solvents were removed by evaporation and crude product was washed with acetone and dried in vacuum. 3-(2,2,2-Trimethylhydrazinium)propionate dihydrate yields are in the range of 90-96% with >99.5% product purity.

In the case of alkyl-3-(2,2,2-trimethylhydrazinium)propionate sulphate or arylalkyl-3-(2,2,2-trimethylhydrazinium)propionate sulphate (or the corresponding hydrogen sulphates), the hydrolysis reaction was carried out by stirring them in the ethanol or another water containing solvent with about equimolar quantity of potassium hydroxide. Depending on the solvent used, reaction temperature can be varied and the corresponding alkanol or arylalkanol, formed as a hydrolysis product, may be simultaneously distilled off the reaction mixture. After the full conversion of the starting materials, controlled chromatographically, reaction mixture was filtered, evaporated and purified, if necessary. Yields of Meldonium were in the range of 90-96%.

Alkylation of the step (a) of the described process proceeds in the range from 50°C to 120°C, preferably in the range from 70°C to 110°C, more preferably in the range from 85°C to 100°C. Hydrolysis of the step (b) of the described process proceeds in the range from 25°C to 100°C, preferably in the range from 70°C to 90 °C.

According to the current invention, methyl-3-(2,2-dimethylhydrazino)propionate is the preferable alkyl-3-(2,2-dimethylhydrazino)propionate for the step (a) of the described process. Alkylation of ethyl- and higher alkyl-3-(2,2-dimethylhydrazino)propionates with DMC in methanol leads to the mixture of products due to the substantive transesterification of the starting propionate. Carrying out the alkylation of higher alkyl esters in corresponding alcohols (e.g., alkylation of ethyl-3-(2,2-dimethylhydrazino)propionate in ethanol) leads to decrease in alkylation reaction rate; elevation of temperature can accelerate the alkylation reaction, but also leads to formation of large amount of side products. Thus, alkylation of ethyl-3-(2,2-dimethylhydrazino)propionate with DMC in ethanol occurs at higher temperature than in the case of methyl ester, and resulting mixture contains more side products. Alkylation of benzyl-3-(2,2-dimethylhydrazino)propionate with DMC in benzyl alcohol at 115°C also gave large amount of side products and only traces of the desired alkylation product. Decrease of alkylation rate and increased formation of side products were observed for ethyl and higher esters both in form of base and sulfate. Alkyl-3-(2,2-dimethylhydrazino)propionate sulphates and arylalkyl-3-(2,2,-dimethylhydrazino)propionate sulphates, prior to the reaction with DMC, were obtained from the corresponding alkyl-3-(2,2-dimethylhydrazino)propionates and aryllkyl-3-(2,2-dimethylhydrazino)propionates by their reaction with sulphuric acid in acetone at room temperature with around 90% yields.

The main advantage of the invented method is the use of a dimethyl carbonate as an alkylating agent for obtaining of the new intermediates for 3-(2,2,2-trimethylhydrazinium)propionate dihydrate preparation. Another advantage of the invented process is the ease of hydrolyzation of the carbonic or sulphuric acid salts of 3-(2,2,2-trimethylhydrazinium)propionate esters, which may proceed in water and/or water-containing solvents, such as lower alkyl alcohols, acetone, ethyl acetate, acetonitrile, dioxane, dimethylformamide, dimethylsulphoxide, alkylketones and mixtures thereof. The method of the present invention overcome the disadvantages of WO 2008/028514 A (SILVA JORGE) 13.03.2008, by providing one step process for obtaining 3-(2,2,2-trimethylhydrazinium)propionate dihydrate from the carbonic and sulphuric acid salts of 3-(2,2,2-trimethylhydrazinium)propionate esters without any additional double salts formation.

Thus, the current invention provide for an inexpensive large scale manufacturing process of 3-(2,2,2-trimethylhydrazinium)propionate dihydrate under use of inexpensive and available reagents.

According to the current invention, alkyl-3-(2,2,2-trimethylhydrazinium)propionate carbonates, hydrogen carbonates, hydrogen sulphates and sulphates are suitable intermediates for producing Meldonium. For this purpose arylalkyl-3-(2,2,2-trimethylhydrazinium)propionate carbonates, hydrogen carbonates, hydrogen sulphates and sulphates, as for example, benzyl-3-(2,2,2-trimethylhydrazinium)propionate carbonates, hydrogen carbonates, hydrogen sulphates and sulphates can be used as well.

Carbonic and sulphuric acid salts of 3-(2,2,2-trimethylhydrazinium)propionate esters can be transferring into 3-(2,2,2-trimethylhydrazinium)propionate dihydrate with high yields. The present invention will be described in more detail by referring to the following non-limiting examples. The scope of the invention should not be limited to the working examples, which are for demonstration purposes. One skilled in the art can practice the invention based on the disclosures in the present patent application.

### Example 1

### Preparation of alkyl-3-(2,2,2-trimethylhydrazinium)propionate or arylalkyl-3-(2,2,2-trimethylhydrazinium)propionate hydrogen carbonates

Alkyl-3-(2,2-dimethylhydrazino)propionate (2 mol) or arylalkyl-3-(2,2-dimethylhydrazino)propionate (2 mol) was dissolved in methanol (300 mL), thereafter dimethylcarbonate (1685 mL, 20 mol) was added to the reaction mixture. Reaction mixture was heated at 90°C in a reactor until the reaction was complete (controlled by GC).

Methanol and dimethyl carbonate were removed by distillation at a reduced pressure. The distillation residue was treated with cooled acetone (~5000 mL). The solid precipitates were filtered; the crude product was washed on the filter with acetone (2000 mL). Yield of the crude alkyl-3-(2,2-dimethylhydrazino)propionate or arylalkyl-3-(2,2,2-trimethylhydrazinium) propionate hydrogen carbonates were in range of 80-96%.

### Example 2

### Preparation of 3-(2,2,2-trimethylhydrazinium)propionate dihydrate

Alkyl-3-(2,2,2-trimethylhydrazinium)propionate hydrogen carbonate or arylalkyl-3-(2,2,2-trimethylhydrazinium) propionate hydrogen carbonate was dissolved in water and reaction mixture was stirred in inert gas atmosphere at 85°C until formation of 3-(2,2,2-trimethylhydrazinium)propionate was complete (controlled by HPLC).

Reaction mixture was evaporated, and then acetone was added. The precipitates were separated by filtration. The obtained 3-(2,2,2-trimethylhydrazinium)propionate dihydrate was dried under reduced pressure. The yield was 90-96%, purity >99.5%.

### Example 3

### Preparation of alkyl-3-(2,2,2-dimethylhydrazino)propionate or arylalkyl-3-(2,2,2-trimethylhydrazinium)propionate carbonates

Alkyl-3-(2,2-dimethylhydrazino)propionate hydrogen carbonate crystalline hydrate or aralkyl-3-(2,2-dimethylhydrazino)propionate hydrogen carbonate crystalline hydrate or their water-free form were heated under vacuum with quantitative formation of the corresponding alkyl-3-(2,2-dimethylhydrazino)propionate or aralkyl-3-(2,2-dimethylhydrazino)propionate carbonates.

### Example 4

### Preparation of 3-(2,2,2-trimethylhydrazinium)propionate dihydrate

Alkyl-3-(2,2,2-trimethylhydrazinium)propionate carbonate or arylalkyl-3-(2,2,2-trimethylhydrazinium)propionate carbonate was dissolved in water and reaction mixture was stirred in inert gas atmosphere at 85°C until formation of 3-(2,2,2-trimethylhydrazinium)propionate was complete (controlled by HPLC).

Reaction mixture was evaporated, and then acetone was added. The precipitates were separated by filtration. The obtained 3-(2,2,2-trimethylhydrazinium)propionate dihydrate was dried at under reduced pressure. The yield was 90-96%, purity >99.5%.

### Example 5

### Preparation of methyl-3-(2,2,2-trimethylhydrazinium)propionate hydrogen carbonate

Methyl-3-(2,2-dimethylhydrazino)propionate (294,4 g, 2 mol) was dissolved in methanol (300 mL), thereafter dimethyl carbonate (1685 mL, 20 mol) was added to the reaction mixture. Reaction mixture was heated at 90°C in a reactor until the reaction was complete (controlled by GC).

Methanol and dimethyl carbonate were removed by distillation at a reduced pressure. The distillation residue was treated with cooled acetone (~5000 mL). The solid precipitates were filtered; the crude product was washed on the filter with acetone (2000 mL). The yield of the crude methyl-3-(2,2,2-trimethylhydrazinium)propionate hydrogen carbonate was 384 g (86.5%).

If methyl-3-(2,2,2-trimethylhydrazinium)propionate hydrogen carbonate was recrystallized from isopropanol, methyl-3-(2,2,2-trimethylhydrazinium)propionate hydrogen carbonate monohydrate was obtained, Mp. 68-70°C, ¹H NMR spectrum (D₂O): 2.63 (2H, t, CH₂COO-); 3.33 (2H, t, NHCH₂CH₂); 3.35 (9H, s, (CH₃)₃N); 3.73 (3H, s, COOCH₃).

### Example 6

### Preparation of methyl-3-(2,2,2-trimethylhydrazinium)propionate hydrogen carbonate

Methyl-3-(2,2-dimethylhydrazino)propionate (294.4 g, 2 mol) was dissolved in methanol (300 mL), thereafter dimethyl carbonate (1685 mL, 20 mol) was added to the reaction mixture. Reaction mixture was stirred at 25°C for 7 days (controlled by GC). Analysis of the reaction mixture shows that reaction does not occur at these conditions.

### Example 7

### Preparation of methyl-3-(2,2,2-trimethylhydrazinium)propionate hydrogen carbonate

Methyl-3-(2,2-dimethylhydrazino)propionate (294.4 g, 2 mol) was dissolved in methanol (300 mL), thereafter dimethyl carbonate (1685 mL, 20 mol) was added to the reaction mixture. Reaction mixture was stirred at 50°C (controlled by GC). After two weeks conversion of methyl-3-(2,2-dimethylhydrazino)propionate is only 2-3%; some impurities also formed.

### Example 8

### Preparation of methyl-3-(2,2,2-trimethylhydrazinium)propionate hydrogen carbonate

Methyl-3-(2,2-dimethylhydrazino)propionate (294.4 g, 2 mol) was dissolved in methanol (300 mL), thereafter dimethyl carbonate (1685 mL, 20 mol) was added to the reaction mixture. Reaction mixture was stirred at 120°C (controlled by GC). After 12 h conversion we observe complete of methyl-3-(2,2-dimethylhydrazino)propionate, but content of desired methyl-3-(2,2,2-trimethylhydrazinium)propionate hydrogen carbonate in the reaction mixture is only few percent, the main products are undefined impurities.

### Example 9

### Preparation of 3-(2,2,2-trimethylhydrazinium)propionate dihydrate

A) Methyl-3-(2,2,2-trimethylhydrazinium)propionate hydrogen carbonate (382g, 1.72 mol) was dissolved in water (2000 mL) and reaction mixture was stirred in inert gas atmosphere at 85°C until formation of 3-(2,2,2-trimethylhydrazinium)propionate was complete (controlled by HPLC).

Reaction mixture was evaporated, and then acetone was added. The precipitates were separated by filtration. The obtained 3-(2,2,2-trimethylhydrazinium)propionate dihydrate was dried under reduced pressure. The yield was 292 g (93.2%, purity >99.5%).

B) Methyl-3-(2,2,2-trimethylhydrazinium)propionate hydrogen carbonate (382g, 1.72 mol) was dissolved in ethanol (4000 mL, 95%) and reaction mixture was stirred in inert gas atmosphere at 85°C until formation of 3-(2,2,2-trimethylhydrazinium)propionate was complete (controlled by HPLC).
Reaction mixture was evaporated, and then acetone was added. The precipitates were separated by filtration. The obtained 3-(2,2,2-trimethylhydrazinium)propionate dihydrate was dried at under reduced pressure. The yield was 298 g (95.1 %, purity >99.5%).

C) Methyl-3-(2,2,2-trimethylhydrazinium)propionate hydrogen carbonate hydrate (240.3 g, 1.0 mol) was dissolved in ethanol (4000 mL, 95%) and reaction mixture was stirred in inert gas atmosphere at 85°C until formation of 3-(2,2,2-trimethylhydrazinium)propionate was complete (controlled by HPLC).
Reaction mixture was evaporated, and then acetone was added. The precipitates were separated by filtration. The obtained 3-(2,2,2-trimethylhydrazinium)propionate dihydrate was dried at under reduced pressure. The yield was 175 g (96.0%, purity >99.5%).

### Example 10

### Preparation of ethyl-3-(2,2,2-trimethylhydrazinium)propionate hydrogen carbonate

Ethyl-3-(2,2-dimethylhydrazino)propionate (320 g, 2 mol) was dissolved in ethanol (300 mL), thereafter dimethyl carbonate (1690 mL, 20 mol) was added to the reaction mixture. Reaction mixture was heated at 115°C in a reactor until the reaction was complete (controlled by GC).

Methanol and dimethyl carbonate were removed by distillation at a reduced pressure. The distillation residue was treated with cooled acetone (~5000 mL). The solid precipitates were filtered; the crude product was washed on the filter with acetone (2000 mL). The yield of crude ethyl-3-(2,2,2-trimethylhydrazinium)propionate hydrogen carbonate was 378 g (80.1 %).
¹H NMR spectrum (D₂O): 1.25 (3H, t, CH₂CH₃); 2.62 (2H, t, CH₂COO); 3.32 (2H, t, NHCH₂CH₂); 3.35 (9H, s, (CH₃)₃N); 4.19 (2H, q, CH₂CH₃).

### Example 11

### Preparation of 3-(2,2,2-trimethylhydrazinium)propionate dihydrate

A) Ethyl-3-(2,2,2-trimethylhydrazinium)propionate hydrogen carbonate (100 g, 0.423 mol) was dissolved in water (500 mL) and reaction mixture was stirred in inert gas atmosphere at 85°C until formation of 3-(2,2,2-trimethylhydrazinium)propionate was complete (controlled by HPLC).

Reaction mixture was evaporated, and then acetone was added. The precipitates were separated by filtration. The obtained 3-(2,2,2-trimethylhydrazinium)propionate dihydrate was dried under reduced pressure. The yield was 71.5 g (92.8%, purity >99.5%).

B) Ethyl-3-(2,2,2-trimethylhydrazinium)propionate hydrogen carbonate (100 g, 0.423 mol) was dissolved in ethanol (1000 mL, 95%) and reaction mixture was stirred in inert gas atmosphere at 85°C until formation of 3-(2,2,2-trimethylhydrazinium)propionate was complete (controlled by HPLC).

Reaction mixture was evaporated, and then acetone was added. The precipitates were separated by filtration. The obtained 3-(2,2,2-trimethylhydrazinium)propionate dihydrate was dried under reduced pressure. The yield was 72.8 g (94.5%, purity >99.5%).

### Example 12

### Preparation of alkyl-3-(2,2,2-dimethylhydrazinium)propionate or arylalkyl-3-(2,2,2-trimethylhydrazinium)propionate hydrogen sulphate

Alkyl-3-(2,2-dimethylhydrazino)propionate sulphate (1.8 mol) or arylalkyl-3-(2,2-dimethylhydrazino)propionate sulphate (1.8 mol) was dissolved in methanol (650 mL), thereafter dimethyl carbonate (1500 mL, 18 mol) was added to the reaction mixture. Reaction mixture was heated at 90°C in a reactor until the reaction was complete (controlled by HPLC).

Methanol and dimethyl carbonate was removed by distillation at a reduced pressure. The distillation residue was treated with cooled acetone (~5000 mL). The reaction mixture was filtered and thereafter filtrate was dissolved in ethanol (300 mL), which was evaporated. The alkyl-3-(2,2,2-dimethylhydrazinium)propionate or arylalkyl-3-(2,2,2-trimethylhydrazinium) propionate hydrogen sulphate was obtained and thereafter dried under reduced pressure. The yield was 60-96%.

### Example 13

### Preparation of 3-(2,2,2-trimethylhydrazinium)propionate dihydrate

Alkyl-3-(2,2,2-dimethylhydrazinium)propionate or arylalkyl-3-(2,2,2-trimethylhydrazinium)propionate hydrogen sulphate was dissolved in ethanol and reaction mixture was stirred in inert gas atmosphere at 0-20 °C at which point potassium hydroxide (1.02 equivalent) solution in ethanol was added dropwise under stirring until formation of 3-(2,2,2-trimethylhydrazinium)propionate was complete (controlled by HPLC).

The precipitates were separated by filtration. The filtrate was heated to 40°C and activated acidic ion exchange resin (Dowex 50WX8, 75 mg) was added to the filtrate and stirred at ambient temperature for 1 hour. The reaction mixture was filtrated and activated charcoal (40 mg) was added and stirred for 15 minutes. Reaction mixture was filtered, evaporated and dried under reduced pressure. The yield of obtained 3-(2,2,2-trimethylhydrazinium)propionate dihydrate was 90-96%.

### Example 14

### Preparation of alkyl-3-(2,2-dimethylhydrazino)propionate sulphates and arylalkyl-3-(2,2,-dimethylhydrazino)propionate sulphates

Alkyl-3-(2,2-dimethylhydrazino)propionate or arylalkyl-3-(2,2,-dimethylhydrazino)propionate (2 mol) were dissolved in acetone and acetone solution of concentrated sulphuric acid (1 mol) was added upon intensive stirring. Reaction mixture was stirred at room temperature for 30 minutes; crude product was filtered, washed with acetone and dried under vacuum. Yields 91-96%.

### Example 15

### Preparation of alkyl-3-(2,2,2-dimethylhydrazinium)propionate or arylalkyl-3-(2,2,2-trimethylhydrazinium) propionate sulphate

Alkyl-3-(2,2-dimethylhydrazino)propionate sulphate (1.8 mol) or arylalkyl-3-(2,2-dimethylhydrazino)propionate sulphate (1.8 mol) was dissolved in ethanol (650 mL), thereafter dimethyl carbonate (1500 mL, 18 mol) was added to the reaction mixture. Reaction mixture was heated at 90°C in a reactor until ester saponification was complete (controlled by HPLC).

Methanol and dimethyl carbonate was removed by distillation at a reduced pressure. The distillation residue was treated with cooled acetone (~5000 mL). The reaction mixture was filtered and thereafter filtrate was dissolved in ethanol (300 mL), which was evaporated. The alkyl-3-(2,2,2-dimethylhydrazinium)propionate or arylalkyl-3-(2,2,2-trimethylhydrazinium)propionate sulphate was obtained and thereafter dried at under reduced pressure. The yield was 60-96%.

### Example 16

### Preparation of methyl-3-(2,2,2-trimethylhydrazinium)propionate sulphate

Methyl-3-(2,2-dimethylhydrazino)propionate sulphate (70.1 g, 0.18 mol) was dissolved in methanol (65 mL), thereafter dimethyl carbonate (150 mL, 1.80 mol) was added to the reaction mixture. Reaction mixture was heated at 90°C in a reactor until the reaction was complete (controlled by HPLC).

Methanol and dimethyl carbonate were removed by distillation at a reduced pressure. The distillation residue was treated with cooled acetone (~5000 mL). The reaction mixture was filtered and thereafter filtrate was dissolved in ethanol (300 mL), which was evaporated. The methyl-3-(2,2,2-trimethylhydrazinium)propionate sulphate was obtained and thereafter dried under reduced pressure. The yield was 61.7 g (82%), ¹H NMR spectrum (D₂O): 2.62 (2H, t, CH₂COO-); 3.32 (2H, t, NHCH₂CH₂); 3.34 (9H, s, (CH₃)₃N); 3.72 (3H, s, COOCH₃).

### Example 17

### Preparation of 3-(2,2,2-trimethylhydrazinium)propionate dihydrate

Methyl-3-(2,2,2-trimethylhydrazinium)propionate sulphate (45.45 g, 0.11 mol) was dissolved in ethanol (25 mL) and reaction mixture was stirred in inert gas atmosphere at 0-20°C at which point potassium hydroxide (14.62 g, 0.22 mol) solution in ethanol (41 mL) was added dropwise and stirred until formation of 3-(2,2,2-trimethylhydrazinium)propionate was complete (controlled by HPLC).

The precipitates were separated by filtration. The filtrate was heated to 40°C and activated acidic ion exchange resin (Dowex 50WX8, 75 mg) was added to the filtrate and stirred at ambient temperature for 1 hour. The reaction mixture was filtrated and activated charcoal (40 mg) was added and stirred for 15 minutes. Reaction mixture was filtered, evaporated to a half volume and cooled to 0°C. The obtained 3-(2,2,2-trimethylhydrazinium)propionate dihydrate was filtered off, washed with cold ethanol and dried under reduced pressure. The yield was 35.6g (90%).

### Example 18

### Preparation of ethyl-3-(2,2,2-trimethylhydrazinium)propionate sulphate

Ethyl-3-(2,2-dimethylhydrazino)propionate sulphate (419 g, 1 mol) was dissolved in methanol (300 mL), thereafter dimethyl carbonate (1690 mL, 20 mol) was added to the reaction mixture. Reaction mixture was heated at 90°C in a reactor until the reaction was complete (controlled by HPLC).

Methanol and dimethyl carbonate were removed by distillation at a reduced pressure. The reaction mixture was filtered and the filtrate evaporated. The distillation residue was treated with acetone (~5000 mL). The precipitated ethyl-3-(2,2,2-trimethylhydrazinium)propionate sulphate was filtered off, washed with acetone and thereafter dried under reduced pressure. The yield was 297 g (61.0 %).

¹H NMR spectrum (D₂O): 1.25 (3H, t, CH₂CH₃); 2.60 (2H, t, CH₂COO); 3.32 (2H, t, NHCH₂CH₂); 3.34 (9H, s, (CH₃)₃N); 4.18 (2H, q, CH₂CH₃).

### Example 19

### Preparation of 3-(2,2,2-trimethylhydrazinium)propionate dihydrate

Ethyl-3-(2,2,2-trimethylhydrazinium)propionate sulphate (179 g, 0.4 mol) was dissolved in ethanol (200 mL) and reaction mixture was stirred in inert gas atmosphere at 0-20°C at which point potassium hydroxide (44.8 g, 0.8 mol) solution in ethanol (200 mL) was added dropwise and stirred until formation of 3-(2,2,2-trimethylhydrazinium)propionate was complete (controlled by HPLC).

The precipitates were separated by filtration. The filtrate was heated to 40°C and activated acidic ion exchange resin (Dowex 50WX8, 7.5 g) was added to the filtrate and stirred at ambient temperature for 1 hour. The reaction mixture was filtrated and activated charcoal (4 g) was added and stirred for 15 minutes. Reaction mixture was filtered, evaporated and dried under reduced pressure Yield of the obtained 3-(2,2,2-trimethylhydrazinium)propionate dihydrate was 134 g (92%).

## Claims

1. A compound of formula (I): 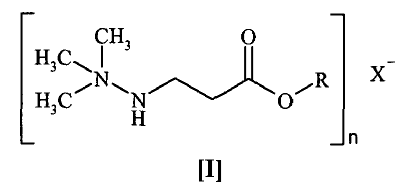 wherein n represents 1 or 2; R represents CH₃, C₂H₅, C₃H₇, i-C₃H₇, C₄H₉, i-C₄H₉ and C₆H₅CH₂; X represents HCO₃ , CO₃²⁻, HSO₄⁻ or SO₄²⁻;

2. A compound according to claim 1, wherein the compound of formula (I) is methyl-3-(2,2,2-trimethylhydrazinium)propionate hydrogen carbonate.

3. A compound according to claim 1, wherein the compound of formula (I) is methyl-3-(2,2,2-trimethylhydrazinium)propionate hydrogen carbonate monohydrate.

4. A compound according to claim 1, wherein the compound of formula (I) is methyl-3-(2,2,2-trimethylhydrazinium)propionate carbonate.

5. A compound according to claim 1, wherein the compound of formula (I) is ethyl-3-(2,2,2-trimethylhydrazinium)propionate hydrogen carbonate.

6. A compound according to claim 1, wherein the compound of formula (I) is benzyl-3-(2,2,2-trimethylhydrazinium)propionate hydrogen carbonate.

7. A compound according to claim 1, wherein the compound of formula (I) is benzyl-3-(2,2,2-trimethylhydrazinium)propionate carbonate.

8. A compound according to claim 1, wherein the compound of formula (I) is methyl-3-(2,2,2-trimethylhydrazinium)propionate hydrogen sulphate.

9. A compound according to claim 1, wherein the compound of formula (I) is methyl-3-(2,2,2-trimethylhydrazinium)propionate sulphate.

10. A compound according to claim 1, wherein the compound of formula (I) is ethyl-3-(2,2,2-trimethylhydrazinium)propionate sulphate.

11. A compound according to claim 1, wherein the compound of formula (I) is benzyl-3-(2,2,2-trimethylhydrazinium)propionate hydrogen sulphate.

12. A compound according to claim 1, wherein the compound of formula (I) is benzyl-3-(2,2,2-trimethylhydrazinium)propionate sulphate.

13. The use of a compound of formula (I) for the synthesis of 3-(2,2,2-trimethylhydrazinium)propionate dihydrate.

14. A process for preparing 3-(2,2,2-trimethylhydrazinium)propionate dihydrate comprising:
a) preparing a compound of formula (I) by reacting the alkyl- or arylalkyl-3-(2,2-dimethylhydrazino)propionate, or its carbonic or sulphuric acid salt with dimethyl carbonate in the presence of alcohol and at a temperature in the range from 50-120°C;
b) hydrolysis the compound of formula (I) prepared in step (a) to produce 3-(2,2,2-trimethylhydrazinium)propionate dihydrate.

15. A process according to claim 14, wherein alcohol in step (a) is selected from the group of methanol or ethanol.

16. A process according to claim 14 wherein the hydrolysis of step (b) proceeds in a water or water containing solvent.

17. A process according to claim 14 wherein the hydrolysis of step (b) proceeds in a solvent, selected from the group, comprising water, lower alcohols, acetone, ethyl acetate, acetonitrile, dioxane, dimethylformamide, dimethylsulphoxide, alkylketones and mixtures thereof.

18. A process according to claim 14 wherein the hydrolysis of step (b) proceeds in basic conditions with pH>7.

19. A process according to claim 14, wherein the compound of formula (I) is methyl-3-(2,2,2-trimethylhydrazinium)propionate carbonate.

20. A process according to claim 14, wherein the compound of formula (I) is ethyl-3-(2,2,2-trimethylhydrazinium)propionate hydrogen carbonate.

21. A process according to claim 14, wherein the compound of formula (I) is benzyl-3-(2,2,2-trimethylhydrazinium)propionate hydrogen carbonate.

22. A process according to claim 14, wherein the compound of formula (I) is benzyl-3-(2,2,2-trimethylhydrazinium)propionate carbonate.

23. A process according to claim 14, wherein the compound of formula (I) is methyl-3-(2,2,2-trimethylhydrazinium)propionate hydrogen sulphate.

24. A process according to claim 14, wherein the compound of formula (I) is methyl-3-(2,2,2-trimethylhydrazinium)propionate sulphate.

25. A process according to claim 14, wherein the compound of formula (I) is ethyl-3-(2,2,2-trimethylhydrazinium)propionate sulphate.

26. A process according to claim 14, wherein the compound of formula (I) is benzyl-3-(2,2,2-trimethylhydrazinium)propionate hydrogen sulphate.

27. A process according to claim 14, wherein the compound of formula (I) is benzyl-3-(2,2,2-trimethylhydrazinium)propionate sulphate.

## Patentansprüche

1. Die Verbindung der Formel (1): 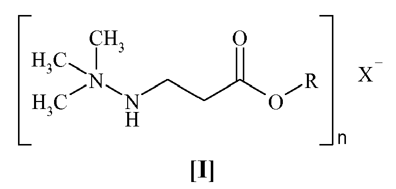 worin n für 1 oder 2 steht; R steht für CH₃, C₂H₅, C₃H₇, i-C₃H₇, C₄H₉, i-C₄H₉ und C₆H₅CH₂, X stellt HCO₃⁻ , CO₃²⁻, HSO₄⁻ or SO₄²⁻ dar;

2. Eine Verbindung nach Anspruch 1, worin die Verbindung der Formel (I) von Methyl-3-(2,2,2-Trimethylhydrazinium)propionat-Hydrogencarbonat ist.

3. Eine Verbindung nach Anspruch 1, worin die Verbindung der Formel (I) von Methyl-3-(2,2,2-Trimethylhydrazinium)propionat-Hydrogencarbonat-Monohydrat ist.

4. Eine Verbindung nach Anspruch 1, worin die Verbindung der Formel (I) von Methyl-3-(2,2,2-Trimethylhydrazinium)propionat-Carbonat ist.

5. Eine Verbindung nach Anspruch 1, worin die Verbindung der Formel (I) von Ethyl-3-(2,2,2-Trimethylhydrazinium)propionat-Hydrogencarbonat ist.

6. Eine Verbindung nach Anspruch 1, worin die Verbindung der Formel (I) von Benzyl-3-(2,2,2-Trimethylhydrazinium)propionat-Hydrogencarbonat ist.

7. Eine Verbindung nach Anspruch 1, worin die Verbindung der Formel (I) von Benzyl-3-(2,2,2-Trimethylhydrazinium)propionat-Carbonat ist.

8. Eine Verbindung nach Anspruch 1, worin die Verbindung der Formel (I) von Methyl-3-(2,2,2-Trimethylhydrazinium)propionat-Hydrogensulfat ist.

9. Eine Verbindung nach Anspruch 1, worin die Verbindung der Formel (I) von Methyl-3-(2,2,2-Trimethylhydrazinium)propionat-Sulfat ist.

10. Eine Verbindung nach Anspruch 1, worin die Verbindung der Formel (I) von Ethyl-3-(2,2,2-Trimethylhydrazinium)propionat-Sulfat ist.

11. Eine Verbindung nach Anspruch 1, worin die Verbindung der Formel (I) von Benzyl-3-(2,2,2-Trimethylhydrazinium)propionat-Hydrogensulfat ist.

12. Eine Verbindung nach Anspruch 1, worin die Verbindung der Formel (I) von Benzyl-3-(2,2,2-Trimethylhydrazinium)propionat-Sulfat ist.

13. Die Verwendung der Verbindung der Formel (I) für die Synthese von 3-(2,2,2-Trimethylhydrazinium)propionat-Dihydrat.

14. Ein Verfahren zur Herstellung von 3-(2,2,2-Trimethylhydrazinium)propionat-Dihydrat, bestehend aus:
a) der Herstellung einer Verbindung der Formel (I) durch die Reaktion von Alkyl- oder Arylalkyl-3-(2,2-Dimethylhydrazin)propionat, oder seinem Kohlensäure- oder Schwefelsäuresalz, mit Dimethyl-Carbonat in Anwesenheit von Alkohol und bei einer Temperatur im Bereich von 50-120°C;
b) der Hydrolyse der Verbindung der Formel (I), die im Schritt (a) vorbereitet wurde, um 3-(2,2,2-trimethylhydrazinium)propionat-Dihydrat herzustellen.

15. Ein Verfahren nach Anspruch 14, worin Alkohol im Schritt (a) aus der Methanol- oder Ethanolgruppe ausgewählt wird.

16. Ein Verfahren nach Anspruch 14, worin die Hydrolyse im Schritt (b) in Wasser oder einem Wasser enthaltendem Lösemittel stattfindet.

17. Ein Verfahren nach Anspruch 14, worin die Hydrolyse im Schritt (b) in einem Lösemittel, das aus der Gruppe ausgewählt wird, bestehend aus Wasser, niedrigen Alkoholen, Aceton, Ethylacetat, Acetonitril, Dioxanen, Dimethylformamid, Dimethylsulfoxid, Alkylketonen und deren Mischungen, stattfindet.

18. Ein Verfahren nach Anspruch 14, worin die Hydrolyse im Schritt (b) unter basischen Bedingungen mit pH>7 stattfindet.

19. Ein Verfahren nach Anspruch 14, worin die Verbindung der Formel (I) von Methyl-3-(2,2,2-Trimethylhydrazinium)propionat-Carbonat ist.

20. Ein Verfahren nach Anspruch 14, worin die Verbindung der Formel (I) von Ethyl-3-(2,2,2-Trimethylhydrazinium)propionat-Hydrogencarbonat ist.

21. Ein Verfahren nach Anspruch 14, worin die Verbindung der Formel (I) von Benzyl-3-(2,2,2-Trimethylhydrazinium)propionat-Hydrogencarbonat ist.

22. Ein Verfahren nach Anspruch 14, worin die Verbindung der Formel (I) von Benzyl-3-(2,2,2-Trimethylhydrazinium)propionat-Carbonat ist.

23. Ein Verfahren nach Anspruch 14, worin die Verbindung der Formel (I) von Methyl-3-(2,2,2-Trimethylhydrazinium)propionat-Hydrogensulfat ist.

24. Ein Verfahren nach Anspruch 14, worin die Verbindung der Formel (I) von Methyl-3-(2,2,2-Trimethylhydrazinium)propionat-Sulfat ist.

25. Ein Verfahren nach Anspruch 14, worin die Verbindung der Formel (I) von Ethyl-3-(2,2,2-Trimethylhydrazinium)propionat-Sulfat ist.

26. Ein Verfahren nach Anspruch 14, worin die Verbindung der Formel (I) von Benzyl-3-(2,2,2-Trimethylhydrazinium)propionat-Hydrogensulfat ist.

27. Ein Verfahren nach Anspruch 14, worin die Verbindung der Formel (I) von Benzyl-3-(2,2,2-Trimethylhydrazinium)propionat-Sulfat ist.

## Revendications

1. Un composé de la formule (I): 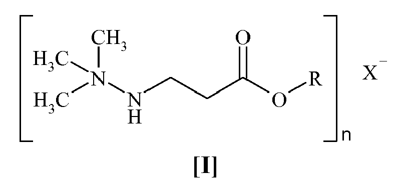 où n représente 1 ou 2; R représente CH₃, C₂H₅, C₃H₇, i-C₃H₇, C₄H₉, i-C₄H₉ et C₆H₅CH₂, X représente HCO₃⁻, CO₃²⁻, HSO₄⁻ or SO₄²⁻;

2. Un composé conformément à l'affirmation 1, où le composé de la formule (I) est le méthyl-3-(2,2,2-triméthylhydrazinium)propionate carbonate d'hydrogène.

3. Un composé conformément à l'affirmation 1, où le composé de la formule (I) est le méthyl-3-(2,2,2-triméthylhydrazinium)propionate carbonate d'hydrogène monohydratée.

4. Un composé conformément à l'affirmation 1, où le composé de la formule (I) est le méthyl-3-(2,2,2-triméthylhydrazinium)propionate carbonate.

5. Un composé conformément à l'affirmation 1, où le composé de la formule (I) est l'éthyl-3-(2,2,2-triméthylhydrazinium)propionate carbonate d'hydrogène.

6. Un composé conformément à l'affirmation 1, où le composé de la formule (I) est le benzyl-3-(2,2,2-triméthylhydrazinium)propionate carbonate d'hydrogène

7. Un composé conformément à l'affirmation 1, où le composé de la formule (I) est le benzyl-3-(2,2,2-triméthylhydrazinium)propionate carbonate.

8. Un composé conformément à l'affirmation 1, où le composé de la formule (I) est le méthyl-3-(2,2,2-triméthylhydrazinium)propionate sulfate d'hydrogène.

9. Un composé conformément à l'affirmation 1, où le composé de la formule (I) est le méthyl-3-(2,2,2-triméthylhydrazinium)propionate sulfate.

10. Un composé conformément à l'affirmation 1, où le composé de la formule (I) est l'éthyl-3-(2,2,2-triméthylhydrazinium)propionate sulfate.

11. Un composé conformément à l'affirmation 1, où le composé de la formule (I) est le benzyl-3-(2,2,2-triméthylhydrazinium)propionate sulfate d'hydrogène.

12. Un composé conformément à l'affirmation 1, où le composé de la formule (I) est le benzyl-3-(2,2,2-triméthylhydrazinium)propionate sulfate.

13. L'utilisation du composé de la formule (I) pour le synthèse de 3-(2,2,2-triméthylhydrazinium)propionate dihydrate.

14. Un procédé pour la préparation de 3-(2,2,2-triméthylhydrazinium)propionate dihydrate comprend:
a) la préparation de la formule (I) en réagissant l'alkyl- ou arylalkyl-3-(2,2-diméthylhydrazino)propionate, ou son sel de l'acide carbonique ou sulfurique avec le diméthyl carbonate en présence de l'alcool et à la température dans le diapason de 50-120°C;
b) hydrolyser le composé de la formule(I) préparé pendant l'étape (a) pour produire 3-(2,2,2-triméthylhydrazinium)propionate dihydrate.

15. Un procédé conformément à l'affirmation 14, où l'alcool pendant l'étape (a) est choisi du groupe de méthanol ou éthanol.

16. Un procédé conformément à l'affirmation 14 où l'hydrolyse de l'étape (b) est procédé dans l'eau ou dans un solvant aquifère.

17. Un procédé conformément à l'affirmation 14 où l'hydrolyse de l'étape (b) est procédé dans un solvant choisi du groupe qui contient l'eau, les alcools faibles, l'acétone, l'acétate d'éthyle, l'acétonitrile, le dioxane, le diméthylformamide, le diméthylsulphoxide, les alkylkétones et leurs mélanges.

18. Un procédé conformément à l'affirmation 14 où l'hydrolyse de l'étape (b) est procédé dans les conditions de base avec le pH>7.

19. Un procédé conformément à l'affirmation 14, où le composé de la formule (I) est le méthyl-3-(2,2,2-triméthylhydrazinium)propionate carbonate.

20. Un procédé conformément à l'affirmation 14, où le composé de la formule (I) est l'éthyl-3-(2,2,2-triméthylhydrazinium)propionate carbonate d'hydrogène.

21. Un procédé conformément à l'affirmation 14, où le composé de la formule (I) est le benzyl-3-(2,2,2-triméthylhydrazinium)propionate carbonate d'hydrogène.

22. Un procédé conformément à l'affirmation 14, où le composé de la formule (I) est le benzyl-3-(2,2,2-triméthylhydrazinium)propionate carbonate.

23. Un procédé conformément à l'affirmation 14, où le composé de la formule (I) est le méthyl-3-(2,2,2-triméthylhydrazinium)propionate sulfate d'hydrogène.

24. Un procédé conformément à l'affirmation 14, où le composé de la formule (I) est le méthyl-3-(2,2,2-triméthylhydrazinium)propionate sulfate.

25. Un procédé conformément à l'affirmation 14, où le composé de la formule (I) est l'éthyl-3-(2,2,2-triméthylhydrazinium)propionate sulfate.

26. Un procédé conformément à l'affirmation 14, où le composé de la formule (I) est le benzyl-3-(2,2,2-triméthylhydrazinium)propionate sulfate d'hydrogène.

27. Un procédé conformément à l'affirmation 14, où le composé de la formule (I) est le benzyl-3-(2,2,2-triméthylhydrazinium)propionate sulfate.
